# EUROPÄISCHE PATENTANMELDUNG

(11) **EP 3 372 665 A1**
(43) Veröffentlichungstag der Anmeldung: **12.09.2018**
(21) Anmeldenummer: 18159624.8
(22) Anmeldetag: 02.03.2018
(51) Int. Cl.: C12M 1/00, C12M 3/06

(54) **VORRICHTUNG, SYSTEM UND VERFAHREN ZUR PRODUKTION PHOTOTROPHER ORGANISMEN**

(30) Priorität: 03.03.2017 DE 102017104439
(71) Anmelder: Technische Hochschule Wildau, 15745 Wildau (DE)
(72) Erfinder: Wildenauer, Franz, 15711 Königs Wusterhausen (DE)
(74) Vertreter: Schulz Junghans Patentanwälte PartGmbB

(57) **Zusammenfassung**

Die Erfindung betrifft eine Vorrichtung, ein System sowie ein Verfahren zur Produktion phototropher Organismen.

Es wird eine Vorrichtung (10) zur Produktion phototropher Organismen zur Verfügung gestellt. Diese umfasst einen Behälter (11), welcher zur Aufnahme einer Flüssigkeit (20) geeignet ist und eine Öffnung (12) aufweist. Die Öffnung (12) ist derart ausgebildet und angeordnet, dass durch die Öffnung (12) von außen in den Behälter (11) einfallendes Licht auf eine Flüssigkeitsoberfläche (22) einer im Behälter (11) angeordneten Flüssigkeit (20) gelangen kann. Die Vorrichtung (10) umfasst wenigstens eine Vibrationseinrichtung (30), welche zur Erzeugung von Turbulenz in der im Behälter (11) angeordneten Flüssigkeit (20) angeordnet und eingerichtet ist.

## Beschreibung

Die Erfindung betrifft eine Vorrichtung, ein System sowie ein Verfahren zur Produktion phototropher Organismen.

Das Wachstum phototropher Organismen in Wasser wird im Wesentlichen vom verfügbaren Licht, Kohlendioxid und Nährstoffen sowie von der Wassertemperatur beeinflusst. Daneben sind Salz- und Sauerstoffzentration sowie der pH-Wert relevant.

Zur Produktion derartiger Organismen, beispielsweise zur Prodiktion von Mikroalgen, werden Photobioreaktoren genutzt, die einen wassergefüllten Behälter umfassen, in welchem die Algen, die genannten Nährstoffe und Kohlendioxid eingebracht werden. Durch direkte oder indirekte Sonneneinstrahlung wird das notwendige Licht zur Realisierung der Photosynthese bereitgestellt. Die effektive Nutzung des zur Verfügung stehenden Lichts ist dabei ein wesentliches Ziel und die Hauptvoraussetzung für einen wirtschaftlichen Prozess.

Die Eindringtiefe des Lichts und damit die Tiefe, in der die Photosynthese möglich ist, ist abhängig von der Biomassekonzentration, also von der Zelldichte, und beträgt typischerweise höchstens wenige Zentimeter. Im Sinne einer hohen Produktivität sind hohe Biomassekonzentrationen wünschenswert, die jedoch den Lichteintrag verringern. Um dennoch einen effektiven Lichtstrom zu den Zellen zu erreichen, werden die bisher bekannten Reaktoren turbulent durchmischt. Damit erfolgt ein Austausch der Wasserschichten, wodurch alle Zellen mit Licht versorgt werden können. Hohe Turbulenzen führen hierbei zu schnellem Zellwachstum.

Durch die Turbulenz wird die Dicke der um die Partikel befindlichen laminaren Grenzschicht komprimiert, welche durch zu transportierende Stoffe überwunden werden muss. Somit werden hohe Konzentrationsgradienten erreicht, die eine hohe Diffusion zur effektiven Versorgung der Zellen mit Nährstoffen und Kohlendioxid sowie zum effektiven Abtransport von Sauerstoff bedingen. Auch der Bewuchs von Oberflächen durch Bildung von Biofilm kann durch Turbulenzen reduziert werden. Weiterhin verhindert die die turbulente Strömung eine Sedimentation der Biomasse.

Bekannte Vorrichtungen zur Produktion phototropher Organismen sind typischerweise als offene Reaktoren, auch als Open Ponds bezeichnet, ausgeführt. Zur Verhinderung der Sedimentation werden Paddelrührwerke eingesetzt, die den Wasserkörper typischerweise mit Strömungsgeschwindigkeiten von 20-30 cm/s laminar bewegen. Zum Einbringen von Turbulenzen werden unterschiedliche Pumpensysteme genutzt, wie beispielsweise Mammutpumpen, Rührwerke, Wasserstrahlpumpen oder Paddelrührwerke. Die typischerweise genutzten offenen Reaktoren haben, bedingt durch die notwendigen Umwälz- bzw. Rührsysteme, eine Höhe von 15-30 cm, was ein geringes Oberflächen-Volumenverhältnis und damit eine geringe Produktivität mit sich bringt.

Wesentlich größere Oberflächen-Volumenverhältnisse und damit wesentlich höhere Produktivitäten werden in horizontalen oder vertikalen Rohr- oder Flachbettreaktoren erzielt. Hierbei sind kurze Lichtwege unter 10 cm möglich. Allerdings ist der Energiebedarf zum Umpumpen oder alternativ zur Durchführung einer Wipp- bzw. Kippbewegung zwecks Erzeugung der benötigten Strömung von 30 cm/s bis 50 cm/s sehr hoch.

Es ist die Aufgabe der Erfindung, eine Vorrichtung, ein System sowie ein Verfahren zur Produktion phototropher Organismen zur Verfügung zu stellen, mit denen auf besonders energieeffiziente und kostengünstige Weise hohe Produktivitäten erzielt werden können. Die Aufgabe wird gelöst durch die Vorrichtung zur Produktion phototropher Organismen gemäß Anspruch 1, das System zur Produktion phototropher Organismen gemäß Anspruch 7 sowie das Verfahren zur Produktion phototropher Organismen gemäß Anspruch 8. Vorteilhafte Ausgestaltungen der Vorrichtung sind in den Unteransprüchen 2-6 angegeben, vorteilhafte Ausgestaltungen des Verfahrens sind in den Unteransprüchen 9 und 10 angegeben.

Ein erster Aspekt der Erfindung ist eine Vorrichtung zur Produktion phototropher Organismen, welche einen Behälter umfasst, der zur Aufnahme einer Flüssigkeit geeignet ist und eine Öffnung aufweist. Die Öffnung ist derart ausgebildet und angeordnet, dass durch die Öffnung von außen in den Behälter einfallendes Licht auf eine Flüssigkeitsoberfläche einer im Behälter aufgenommenen Flüssigkeit gelangen kann. Weiterhin umfasst die Vorrichtung wenigstens eine Vibrationseinrichtung, welche zur Erzeugung von Turbulenz in der im Behälter aufgenommenen Flüssigkeit angeordnet und eingerichtet ist.

Phototrophe Organismen, beispielsweise aquatische Mikroorganismen, sind Organismen, die in der Lage sind, Licht als Energiequelle zu nutzen. Insbesondere sind in Zellkultur kultivierbare aquatische Mikroorganismen gemeint, wie beispielsweise einzellige Grünalgen wie: *Chlorella spec., Scenedesmus spec., Pediasturm spec., Dunaliella spec.* oder Haematococcus spec., oder Cyanobakterien wie: *Spirulina spec.* oder *Nostoc spec.,* oder Halobakterien wie: *Halobacterium spec.*

Typischerweise ist der Behälter nach oben hin offen. Die Flüssigkeit kann gelöste Stoffe enthalten und/oder eine Dispersion aus festen, flüssigen und/oder gasförmigen Stoffen sein. Die Vibrationseinrichtung ist insbesondere dazu eingerichtet, in der im Behälter aufgenommenen Flüssigkeit dauerhaft und im Wesentlichen vollständig eine Turbulenz zu erzeugen. Zu diesem Zweck können auch mehrere Vibrationseinrichtungen angeordnet sein. Insbesondere wird das Erzeugen von Turbulenz mittels Einbringung von Schwingungen in die Flüssigkeit realisiert.

Turbulenz meint Verwirbelungen in der Flüssigkeit. Das Erzeugen von Turbulenz kann dabei, insbesondere bei fließenden Flüssigkeiten, durch das Einstellen bzw. Erreichen von Reynolds-Zahlen oberhalb der kritischen Reynolds-Zahl gekennzeichnet sein. Insbesondere ist die Vibrationseinrichtung eingerichtet, Vibrationen in einem FrequenzBereich von 600 bis 9000 min⁻¹ mit einer Amplitude von 0,2-11 mm in der Flüssigkeit zu erzeugen.

Durch die einbringbare Turbulenz können in einer im Behälter aufgenommenen Flüssigkeit vorhandene Partikel, also auch enthaltene Organismen, in der Schwebe und damit in Bewegung gehalten werden. Bei durchströmten Behältern wird eine turbulente Strömung erzeugt und somit eine laminare Strömung verhindert, insbesondere an der Flüssigkeitsoberfläche. Somit kann einfallendes Licht eine maximale Zahl von Organismen erreichen und so die Wachstumsgeschwindigkeit der Organismen deutlich erhöhen. Weiterhin werden die Sedimentation der Organismen und das Anwachsen von Biomasse an der Behälteroberfläche bzw. an Einbauten verhindert. Somit wird ein stabiler Prozess mit konstanten Bedingungen im gesamten Behältervolumen ermöglicht.

Neben dem geringen Energieverbrauch zur Vermeidung der Sedimentation und Bewerkstelligung des turbulenten Austausches ist ein weiterer wichtiger Vorteil der mittels der Vibrationseinrichtung erzeugten Turbulenz die geringe Scherbelastung. Viele Algenarten sind empfindlich gegen Scherkräfte, die vor allem durch Pumpen erzeugt werden. Dadurch, dass die notwendige Energie über eine große Fläche, beispielsweise über den gesamten Behälterboden, eingetragen werden kann, ist die Scherbelastung extrem gering. Insbesondere ist die Vibrationseinrichtung zur lokalen Schwingungserzeugung eingerichtet. Sie kann eine mechanisch mit der Flüssigkeit in Kontakt bringbare Vibrationseinrichtung sein, beispielsweise umfassend ein Schwingelement.

In einer Ausgestaltung umfasst die Vibrationseinrichtung ein Schwingelement sowie einen damit mechanisch verbundenen oder verbindbaren maschinellen Antrieb, welcher dazu eingerichtet ist, eine Bewegung des Schwingelementes zu realisieren.

Demzufolge ist die Vibrationseinrichtung in direkten oder indirekten Kontakt mit der Flüssigkeit bringbar. Insbesondere ist die erzeugte Bewegung eine Schwingung. Sie kann beispielsweise eine lineare Schwingung oder eine Drehschwingung sein.

Ein Schwingelement im Sinne der Erfindung ist ein Element, welches zur Erzeugung von Schwingungen eingerichtet ist. Dabei muss das Schwingelement selbst nicht notwendigerweise zur Durchführung einer Schwingung eingerichtet sein und seine realisierte Bewegung muss ebenfalls nicht notwendigerweise eine Schwingung sein. Das Schwingelement kann ein exzentrisch rotierbares Element umfassen und/oder zur Realisierung einer Drehschwingung eingerichtet sein. Auch ein pneumatischer Kolben kann als Schwingelement genutzt werden. Ein Schwingelement kann dabei eine oder mehrere mechanische Verbindungen zum Behälter zur Übertragung von Bewegungen auf den Behälter zwecks Erzeugung von Turbulenz in der Flüssigkeit aufweisen.

Der maschinelle Antrieb ist insbesondere ein elektrischer bzw. elektromechanischer Antrieb, z.B. ein Elektromotor, welcher dazu eingerichtet ist, bei Anlegen eines elektrischen Stroms eine Bewegung des Schwingelementes zu erzeugen. Daneben können auch alle anderen maschinellen Antriebe genutzt werden.

Alternativ kann die Vibrationseinrichtung eine Ultraschalleinrichtung zur mechanisch verbundenen oder berührungslosen Erzeugung von Turbulenzen aufweisen.

In Vergleich zu den üblicherweise genutzten Paddelrührwerken, die eine laminare Strömung der Flüssigkeit erzeugen, wird mit der erfindungsgemäßen Vorrichtung ein wesentlich effizienterer Prozess zur Produktion phototropher Organismen möglich, da nicht der gesamte Flüssigkeitskörper umgewälzt werden muss, der Lichtstrom hin zu den einzelnen Organismen dagegen vergleichbar hoch bleibt. Es sind bei gleicher Produktion von Organismen signifikant geringere Bauhöhen der Reaktoren möglich, da für das Funktionieren der Vibrationseinrichtung keine Mindesthöhe der Flüssigkeit eingehalten werden muss, wie es bei Paddelrührwerken der Fall ist. Die Photosynthese läuft, wie beschrieben, bedingt durch die Abschwächung des Lichteinfalls mit der Eindringtiefe des Lichts in aller Regel nur in einem kleinen oberflächennahen Bereich, z.B. im obersten Zentimeter, effizient ab. Die erzeugte Turbulenz ermöglicht einen effizienten Austausch zwischen beleuchteten und unbeleuchteten Bereichen und damit eine gute und gleichmäßige Beleuchtung aller im Behälter enthaltenen Mikroorganismen. Auch im Vergleich zu den ebenfalls herkömmlich genutzten Umwälzpumpen ist der Energieeintrag signifikant geringer, da auch hier kein Umwälzen des Wasserkörpers zur effizienten Beleuchtung der Organismen notwendig ist. Durch die effiziente Erzeugung von Turbulenz ist eine große Zelldichte, also eine hohe Biomassekonzentration möglich, was wiederum den absoluten Massenzuwachs an Biomasse erhöht.

In einer Ausgestaltung der Vorrichtung ist die Vibrationseinrichtung zur Übertragung von Vibrationen auf die Flüssigkeit eingerichtet, insbesondere mittels eines zur Übertragung von Vibrationen auf die Flüssigkeit mechanisch mit der Vibrationseinrichtung gekoppelten Übertragungselements und/oder unter Verwendung eines maschinellen Antriebs.

Dies ist mit in die Flüssigkeit zumindest teilweise eingetauchten bzw. eintauchbaren Vibrationseinrichtungen möglich. Alternativ kann wenigstens ein Übertragungselement angeordnet sein, also eine Einrichtung, die mechanisch mit der Vibrationseinrichtung verbunden sind und wenigstens teilweise in die Flüssigkeit ragen. Derartige Elemente können auch als Einbauten bezeichnet werden und weisen typischerweise eine von der Flüssigkeit kontaktierte bzw. kontaktierbare Oberfläche auf. Dabei kann insbesondere ein Schwingelement mit dem Übertragungselement mechanisch gekoppelt sein.

In dieser Ausgestaltung kann der maschinelle Antrieb als Elektromotor realisiert und dazu eingerichtet sein, bei Anlegen eines elektrischen Stroms eine Bewegung des Übertragungselements zu erzeugen.

In einer weiteren Ausgestaltung weist der Behälter einen im Wesentlichen ebenen Behälterboden mit einer zur Behälterinnenseite weisenden Oberfläche auf und die Vorrichtung weist eine Aufstelleinrichtung zum Aufstellen des Behälters auf. Dabei ist die Aufstelleinrichtung dazu eingerichtet, den Behälter derart auf einer Aufstellebene zu positionieren, dass die zur Behälterinnenseite weisende Oberfläche parallel zur Aufstellebene oder in einem Winkel zwischen 0,1 Grad und 5 Grad und insbesondere zwischen 0,5 Grad und 3 Grad in Bezug zur Aufstellebene angeordnet ist. In einer Ausgestaltung beträgt der Winkel zur Aufstellebene ca. 2 Grad.

Insbesondere ist die Aufstellebene horizontal, so dass ein horizontaler oder leicht bezüglich der Horizontalen geneigter innerer Behälterboden zur Verfügung gestellt wird.

Dies ermöglicht, dass sich eine im Behälter aufgenommene Flüssigkeit auf einer parallel oder winklig zur Aufstellebene positionierten und im Wesentlichen ebenen Oberfläche des Behälterbodens befindet.

Die Aufstelleinrichtung kann beispielsweise ein Gestell und/oder Stützelemente umfassen, welche geeignet sind, den Behälter wie beschrieben zu positionieren. Sie kann auch durch einen unteren Bereich des Behälterbodens ausgebildet sein, welcher winklig oder parallel zum oberen Bereich des Behälterbodens bzw. zu der zur Behälterinnenseite weisenden Oberfläche ausgerichtet ist.

Mit anderen Worten umfasst die erfindungsgemäße Vorrichtung einen ebenen Behälter bzw. einen leicht geneigten Behälter zur Realisierung einer Strömung der im Behälter befindlichen Flüssigkeit.

In einer weiteren Ausgestaltung weist der Behälter einen im Wesentlichen ebenen Behälterboden sowie wenigstens ein Wandungselement zwecks Rückhalts der Flüssigkeit im Behälter auf. Dabei weist das Wandungselement eine rechtwinklig zum Behälterboden gemessene Höhe H auf, die zwischen 0,5 cm und 15 cm und insbesondere zwischen 1 cm und 5 cm beträgt.

Mit anderen Worten weisen die Flüssigkeitsoberfläche einer im Behälter aufgenommenen Flüssigkeit, also ein sich einstellender Flüssigkeitsspiegel, und der Behälterboden einen mittleren Abstand der angegebenen Größe auf. Insbesondere kann das durch Ausbildung von Behälterwandungen realisiert werden, die im Wesentlichen die Höhe H aufweisen. Die Höhe H wird dabei von der mittleren Höhe des Behälterbodens aus gemessen. Typischerweise sind umlaufend Wandungselemente angeordnet.

Als Wandungselement wird jede Erhebung von dem im Wesentlichen ebenen Behälterboden bezeichnet, die zum Rückhalt einer Flüssigkeit geeignet ist. Wandungselemente können einteilig mit dem Behälterboden, beispielsweise durch Umformprozesse, hergestellt werden. Typischerweise umfasst der Behälter dagegen einen flachen Boden und daran im Wesentlichen senkrecht angeschlossene, beispielsweise angeschweißte, Wandungen.

In einer Ausgestaltung weisen die Flüssigkeitsoberfläche und der Behälterboden einen Abstand von weniger als 10 cm auf.

Mit anderen Worten erstreckt sich die Wandung des Behälters nicht höher als 15 cm und insbesondere nicht höher als 10 cm über die mittlere Höhe des Behälterbodens hinaus. Es wird somit ein Flachbettreaktor zur Verfügung gestellt.

Die Flüssigkeitsoberfläche kann durch eine ruhende oder bewegte Flüssigkeit ausgebildet sein. Insbesondere bei Einstellung eines Winkels zur Aufstellebene, wie beschrieben, ist die im Behälter aufgenommene Flüssigkeit ein fließender Flüssigkeitsfilm.

Die erfindungsgemäße Vorrichtung ist durch ihr großes Oberflächen-Volumenverhältnis in der Lage, signifikant höhere Biomasse-Produktivitäten zu erzielen als die aus dem Stand der Technik bekannten Vorrichtungen.

Zumindest ein Teil des Behälters kann als Übertragungselement ausgestaltet sein. Insbesondere kann der Behälter einen Behälterboden aufweisen und der Behälterboden als Übertragungselement ausgestaltet sein.

Die Vibrationseinrichtung kann demnach mechanisch mit dem Behälter gekoppelt sein und dazu eingerichtet sein, Vibrationen in den Behälter einzubringen. Dabei kann die Vibrationseinrichtung mechanisch mit dem Behälterboden gekoppelt sein und dazu eingerichtet sein, Vibrationen in diesen einzubringen.

In dieser Ausgestaltung werden Vibrationen nicht unmittelbar von der Vibrationseinrichtung auf die Flüssigkeit übertragen.

Gleichzeitig oder alternativ dazu kann die Vibrationseinrichtung dazu eingerichtet sein, wenigstens teilweise in die Flüssigkeit eingetaucht zu werden und Vibrationen auf die Flüssigkeit zu übertragen.

Weiterhin kann der Behälter wenigstens ein Wandungselement aufweisen und die Vibrationseinrichtung mechanisch mit dem Wandungselement gekoppelt sein und dazu eingerichtet sein, Vibrationen in das Wandungselement einzubringen.

In einer weiteren Ausgestaltung umfasst die Vorrichtung zur Produktion phototropher Organismen weiterhin einen Speicherbehälter zum Zwischenspeichern von Flüssigkeit sowie wenigstens eine geeignete Fließverbindung zum Transport von Flüssigkeit vom Behälter in den Speicherbehälter und zurück.

Insbesondere umfasst die Vorrichtung wenigstens eine geeignete Fördereinrichtung, insbesondere eine Pumpe, zum Antrieb der Flüssigkeit zwecks Transports der Flüssigkeit vom Behälter in den Speicherbehälter bzw. vom Speicherbehälter in den Behälter. Die Fließverbindung kann dabei eine Schlauch- oder Rohrleitung oder eine offene Verbindung sein, beispielsweise umfassend einen Überlauf und einen Bereich zum schwerkraftbedingten Überlaufen.

Der Speicherbehälter kann eine Wärmeisolierung und/oder eine Einrichtung zum Temperieren der Flüssigkeit aufweisen. Insbesondere weist er eine Erwärmungs- bzw. Warmhalteeinrichtung auf.

Der Speicherbehälter ist insbesondere derart dimensioniert, dass in kurzer Zeit, also in weniger als einer Stunde und insbesondere weniger als dreißig Minuten die gesamte Flüssigkeitsmenge vom Behälter in den Speicherbehälter bzw. vom Speicherbehälter in den Behälter transportiert werden kann.

Ein zweiter Aspekt der Erfindung ist ein System zur Produktion phototropher Organismen. Dieses umfasst die erfindungsgemäße Vorrichtung zur Produktion phototropher Organismen sowie eine darin aufgenommene Flüssigkeit, wobei die Flüssigkeit im Wesentlichen Wasser oder im Wesentlichen ein Wasser-Biomasse-Gemisch ist. Insbesondere ist die Vibrationseinrichtung dazu eingerichtet und angeordnet, zumindest Teile des Systems zur Schwingung in ihrem Eigenfrequenzbereich ω₀ᵣ oder mit einer Erregerfrequenz fᵣ anzuregen, wobei gilt: fᵣ = ω₀ᵣ * 2ⁿ; wobei n eine ganze Zahl sein soll.

Die Flüssigkeit kann gelöste Stoffe enthalten und eine Dispersion sein, wie zum Beispiel eine Suspension von Biomasse und/oder anderen partikulären Stoffen in Wasser.

Insbesondere enthält das Wasser Nährstoffe bzw. Nährsalze und/oder es können derartige Stoffe hinzugefügt werden, wie beispielsweise Ammonium, Nitrat, Phosphat Kalium, Molybdän und/oder weitere Spurenelemente. Weiterhin kann das Wasser Hilfsstoffe enthalten, beispielsweise wachstumshemmende Stoffe wie Antibiotika oder Schwermetallsalze, beispielsweise Kupfer und/oder Zink. Auch können dem Wasser Partikel zugegeben werden, die mittels ihrer abrasiven Eigenschaften einen Bewuchs von Anlagenteilen durch Bildung eines Biofilms verhindern. Diese können beispielsweise aus Kunststoff, Gestein oder Metall bestehen und/oder eine ähnliche Dichte wie Wasser haben. Im Wesentlichen Wasser bedeutet, dass der größte Anteil der Flüssigkeit Wasser ist. Beispielsweise können mehr als 75 Massenprozent und insbesondere mehr als 90 Massenprozent Wasser sein.

Durch die Anregung von zumindest Teilen des Systems in deren Eigenfrequenz und/oder einem entsprechenden Bruchteil bzw. Vielfachen der Eigenfrequenz entstehen besonders große Amplituden bzw. es lassen sich, bedingt durch die Resonanz, bei niedrigem Energieeinsatz besonders effizient Schwingungen realisieren. Mit anderen Worten wird eine Frequenz gewählt, die einer Resonanzfrequenz oder einem Bruchteil bzw. einem ganzzahligen Vielfachen dieser von zumindest Teilen des Systems entspricht. Eine ganze Zahl kann positiv oder negativ sein. Insbesondere sind folgende Faktoren der Eigenfrequenz nutzbar: 1/8, ¼, ½, 2, 4, 8.

Für eine geeignete Lagerung ist selbstverständlich zu sorgen, damit es durch die Schwingungen nicht zur Zerstörung des Systems kommt.

Ein dritter Aspekt der Erfindung ist ein Verfahren zur Produktion phototropher Organismen. Dieses umfasst die Schritte Bereitstellung eines erfindungsgemäßen Systems zur Produktion phototropher Organismen, wobei phototrophe Organismen, insbesondere aquatische Mikroorganismen, sowie insbesondere Nährstoffe in der Flüssigkeit angeordnet sind. Es erfolgt das Erzeugen von Turbulenz in der Flüssigkeit mittels der Vibrationseinrichtung, sowie das Einbringen von Licht von außen durch die Öffnung des Behälters auf die Flüssigkeitsoberfläche.

Organismen, Nährstoffe und/oder weitere Stoffe können in der Flüssigkeit schon zuvor vorhanden sein oder zugegeben werden. Das Erzeugen von Turbulenz und das Einbringen von Licht können zumindest zeitabschnittsweise zeitgleich erfolgen. Das Einbringen von Licht meint insbesondere das Einfallenlassen von Tageslicht durch die Öffnung des Behälters auf die Wasseroberfläche. Das einfallende Licht kann die Flüssigkeit dabei teilweise oder vollständig durchdringen.

Zur Erzeugung von Turbulenz in der Flüssigkeit kann die Vibrationseinrichtung beispielsweise eine Schwingungsbewegung mit einer Amplitude im Bereich von 0,5mm bis 5 mm und insbesondere im Bereich von 0,75mm bis 1,5mm erzeugen.

An Stelle der Bereitstellung eines erfindungsgemäßen Systems zur Produktion phototropher Organismen kann eine ebenso erfindungsgemäße Vorrichtung zur Produktion phototropher Organismen zur Verfügung gestellt und mit einer Flüssigkeit gefüllt werden, die im Wesentlichen Wasser ist und insbesondere Nährstoffe enthält.

In einer Ausgestaltung des Verfahrens wird Kohlendioxid oder ein Kohlendioxid enthaltendes Gasgemisch, insbesondere ein Abgas aus einem Verbrennungsprozess, in die Flüssigkeit eingebracht und/oder auf die Flüssigkeit gegeben.

Derartige Abgase fallen bei einer Vielzahl von Verbrennungsprozessen an, beispielsweise in Blockheizkraftwerken, und werden hinsichtlich des Kohlendioxids zumeist nicht stofflich verwertet.

Beispielsweise wird oberhalb des Flüssigkeitsspiegels mittels geeigneter Einrichtungen wie durchsichtiger Folien oder Platten eine zumindest im Wesentlichen gasdichte Atmosphäre erzeugt, in die das Kohlendioxid oder das Kohlendioxid enthaltende Gasgemisch eingebracht wird, so dass es zumindest teilweise in der Flüssigkeit gelöst wird. Alternativ kann ein Teilstrom oder der gesamte Strom der Flüssigkeit entnommen werden und mit dem Kohlendioxid oder dem Kohlendioxid enthaltenden Gasgemisch angereichert werden, beispielsweise mittels eines geeigneten Injektors oder einer Dosierlanze. Auch kann im Behälter eine geeignete Begasungseinrichtung zum Einblasen des Gases bzw. Gasgemischs angeordnet werden.

Neben der Bereitstellung des Kohlendioxids als für das Wachstum der Mikroorganismen notwendigen Stoffs kann dieses auch zur Steuerung des pH-Wertes der Flüssigkeit genutzt werden.

Wird Abgas aus einem Verbrennungsprozess genutzt, welches eine höhere Temperatur aufweist als die im Behälter aufgenommene Flüssigkeit, kann dieses zusätzlich zur Übertragung von Wärme zwecks Erwärmung der Flüssigkeit bzw. Einstellung eines für das Wachstum der Mikroorganismen geeigneten Temperaturbereichs genutzt werden.

In einer weiteren Ausgestaltung des Verfahrens wird wenigstens ein Teil der Flüssigkeit und insbesondere die gesamte Flüssigkeit bewegt, so dass sich ein Flüssigkeitsstrom im Behälter einstellt, wobei insbesondere die Geschwindigkeit der Bewegung des Flüssigkeitsstroms maximal 0,5 m/s beträgt.

Mit der Bewegung der Flüssigkeit ist hierbei nicht die Bewegung gemeint, die durch die Vibrationseinrichtung, also über Turbulenzen, in der Flüssigkeit hervorgerufen wird. Insbesondere erfolgt die Bewegung der Flüssigkeit mittels Pumpen.

Auf diese Weise wird die Schichtung des Wassers aufgebrochen und eine gleichmäßige Verteilung der Biomasse bzw. eine gleichmäßige und effektive Beleuchtung aller Mikroorganismen erreicht. Auch werden die Sedimentation partikulärer Stoffe wie Mikroorganismen und das Aufwachsen von Biomasse an Teilen des Systems weiter verringert. Es kann eine turbulente oder laminare Strömung erzeugt werden, wobei mittels der Vibrationseinrichtung Turbulenzen erzeugt werden.

Insbesondere wird die Flüssigkeit in einem wenigstens teilgeschlossenen Kreislauf bewegt, also gänzlich oder teilweise im Kreis gefördert, insbesondere gepumpt. Dabei können Teilströme der Flüssigkeit und/oder weiterer Stoffe hinzu- und/oder abgeleitet werden.

In einer Ausgestaltung des Verfahrens weist der Behälter einen im Wesentlichen ebenen, geneigten Behälterboden auf und es wird wenigstens ein Teil der Flüssigkeit und insbesondere die gesamte Flüssigkeit bewegt, so dass sich ein Flüssigkeitsstrom auf dem Behälterboden einstellt. Dabei beträgt insbesondere die Geschwindigkeit der Bewegung des Flüssigkeitsstroms maximal 0,5 m/s. Die Flüssigkeit wird dabei insbesondere in einem Kreislauf bewegt.

Dies kann mittels einer Pumpe realisiert werden, die beispielsweise mechanisch und/oder hydraulisch wirken kann und mit entsprechenden Rohrleitungen an den Behälter angebunden ist. Die Neigung des Behälterbodens kann dabei eine Strömung bzw. eine Bewegung der Flüssigkeit bedingen und/oder verstärken. Insbesondere ist der Flüssigkeitsspiegel in diesem Fall im Wesentlichen parallel zum Behälterboden.

Es kann an der tieferliegenden Seite des Behälters bzw. an der tiefsten Stelle der Behälterwandung ein Überlauf bzw. eine Überfallkante angeordnet sein, über die die Flüssigkeit aus dem Behälter ausströmt. Die Flüssigkeit wird anschließend zurück zum höher liegenden Teil des Behälters gefördert, um erneut einen Flüssigkeitsstrom in dem Behälter zu erzeugen. Dabei kann auch Kohlendioxid der Flüssigkeit zugegeben werden, beispielsweise mittels Einblasen von Kohlendioxid oder eines Kohlendioxid enthaltenden Gasgemischs in der Rohrleitung.

Die Höhe der Wasserschicht im Behälter beträgt beispielsweise maximal 10 cm und insbesondere maximal 5 cm, was z. B. durch ein Wandungselement mit einer rechtwinklig zum Behälterboden gemessenen Höhe H auf, die in diesem Bereich liegt. Mit anderen Worten wird ein Flachbett-Prozess zur Verfügung gestellt, welcher bei wesentlich geringerem Bauvolumen und Energieeinsatz eine vergleichbare Biomasse-Produktivität zur Verfügung stellt wie ein herkömmlicher Prozess.

In einer weiteren Ausgestaltung des Verfahrens umfasst die genutzte Vorrichtung zur Produktion phototropher Organismen einen Speicherbehälter zum Zwischenspeichern von Flüssigkeit sowie eine geeignete Fließverbindung zum Transport von Flüssigkeit vom Behälter in den Speicherbehälter und zurück. Es wird wenigstens ein Teil der Flüssigkeit und insbesondere die gesamte Flüssigkeit mittels der Fließverbindung vom Behälter in den Speicherbehälter gefördert. Dies kann insbesondere beim Auftreten definierter Randbedingungen erfolgen. Derartige Randbedingungen können eine definierte Zeit, ein Auftreten starker Niederschläge oder eine geringe auf den Flüssigkeitsspiegel eintreffenden Lichtmenge sein, beispielsweise bei Anbruch der Nacht. Die Flüssigkeit kann im Behälter erwärmt werden bzw. ihre Temperatur kann konstant gehalten werden. Anschließend wird wenigstens ein Teil der Flüssigkeit und insbesondere die gesamte Flüssigkeit mittels der Fließverbindung vom Speicherbehälter zurück in den Behälter gefördert. Dies kann ebenso beim Auftreten definierter Randbedingungen erfolgen, wie einer definierten Zeit, einem Ende eines starken Niederschlags oder einer hohen Lichtmenge am bzw. im Behälter, beispielsweise bei Anbruch des Tages.

Die Erfindung wird im Folgenden anhand des in der beiliegenden Zeichnung dargestellten Ausführungsbeispiels erläutert.

### Es zeigt

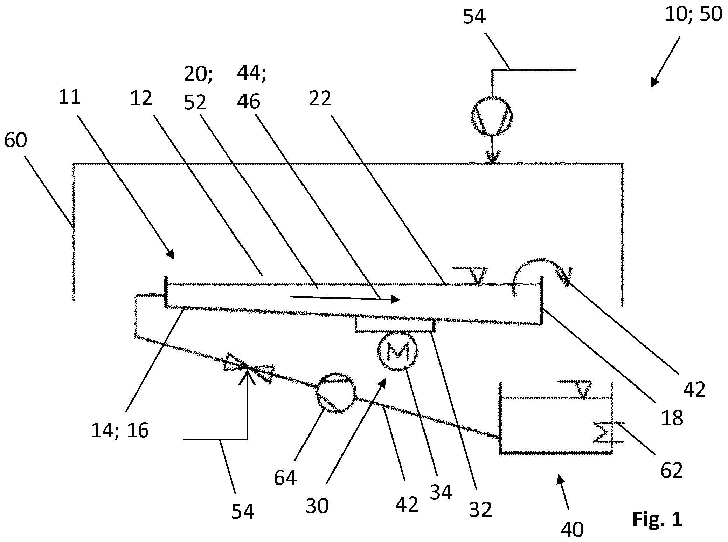

Fig. 1: eine schematische Seitenansicht eines erfindungsgemäßen Systems zur Produktion phototropher Organismen.

Gezeigt ist ein System 50 zur Produktion phototropher Organismen in Gestalt einer mit einer Flüssigkeit 20, nämlich einem Wasser-Biomasse-Gemisch 52, gefüllten Behälters 11 einer Vorrichtung 10 zur Produktion phototropher Organismen. Der Behälter 11 ist nach oben hin vollständig geöffnet, weist also eine oben liegende Öffnung 12 auf. Er umfasst einen Behälterboden 14 mit einer nach oben weisenden, im Behälter 11 liegenden Oberfläche 16. An den Behälterboden 14 angeschlossen sind rechts und links dargestellte Wandungselemente 18. Diese weisen eine Höhe H von 10 cm auf, so dass die Flüssigkeitsoberfläche 22 der im Behälter 11 befindlichen Flüssigkeit 20 maximal 10 cm über dem Behälterboden 14 liegt.

An der Unterseite des Behälterbodens 14 ist eine Vibrationseinrichtung 30 angeordnet. Diese umfasst ein Übertragungselement 32 und einen mechanisch damit verbundenen maschinellen Antrieb 34 zur Realisierung von Vibrationen und zur Übertragung auf das Übertragungselement 32. Das Übertragungselement 32 dient der Übertragung der Vibrationen auf den eben ausgeführten Behälterboden 14 zwecks Erzeugung von Turbulenz in der Flüssigkeit 20. Somit ist auch die Vibrationseinrichtung 30 mechanisch mit dem Behälterboden 14 gekoppelt.

Die Vibrationseinrichtung 30 ist dazu eingerichtet und angeordnet, den Behälterboden 14 des Systems 50 zur Schwingung in seiner Eigenfrequenz anzuregen.

Neben dem Behälter 11 ist ein Speicherbehälter 40 dargestellt, der mittels zweier Fließverbindungen 42 mit dem Behälter 11 verbunden ist. Zum einen ist ein rechts dargestelltes Wandungselement 18 des Behälters 11 als Überfallkante ausgeführt, über welches die Flüssigkeit 20 schwerkraftbedingt in den nach oben hin geöffneten Speicherbehälter 40 fließen kann. Zum anderen ist zwischen Speicherbehälter 40 und Behälter 11 als Fließverbindung 42 eine Rohrleitung mit einer Pumpe 64 angeordnet, um die Flüssigkeit zurück in den Behälter 11 zu transportieren. In Kombination mit der winklig angeordneten, im Behälter 11 liegenden Oberfläche 16 des Behälterbodens 14 kann auf diese Weise eine Bewegung 46 der im Behälter 11 befindlichen Flüssigkeit in Form eines turbulenten Flüssigkeitsstroms 44 realisiert werden.

Im Speicherbehälter 40 ist eine Heiz-/Kühleinrichtung zur Temperierung der Flüssigkeit 20 angeordnet.

Oberhalb des Behälters 11 ist eine mittels einer Folie 60 erzeugte gasdichte Atmosphäre angeordnet, in die Kohlendioxid 54 eingeleitet wird. Dieses löst sich im Wasser-Biomasse-Gemisch 52 und wird somit den phototrophen Organismen zur Verfügung gestellt. Alternativ oder zusätzlich zur Begasung der Flüssigkeitsoberfläche 22 kann das Kohlendioxid 54 über ein Ventil, welches in der als Rohrleitung ausgestalteten Fließverbindung 42 zwischen Speicherbehälter 40 und Behälter 11 liegt, eingeleitet und gelöst werden.

### Bezugszeichenliste

- Vorrichtung: 10
- Behälter: 11
- Öffnung: 12
- Behälterboden: 14
- Oberfläche: 16
- Wandungselement: 18
- Aufstelleinrichtung: 19
- Flüssigkeit: 20
- Flüssigkeitsoberfläche: 22
- Vibrationseinrichtung: 30
- Übertragungselement: 32
- Maschineller Antrieb: 34
- Speicherbehälter: 40
- Fließverbindung: 42
- Flüssigkeitsstrom: 44
- Bewegung: 46
- System: 50
- Wasser-Biomasse-Gemisch: 52
- Kohlendioxid: 54
- Folie: 60
- Heiz-/Kühleinrichtung: 62
- Pumpe: 64

## Patentansprüche

1. Vorrichtung (10) zur Produktion phototropher Organismen, umfassend einen Behälter (11), welcher zur Aufnahme einer Flüssigkeit (20) geeignet ist und eine Öffnung (12) aufweist, wobei die Öffnung (12) derart ausgebildet und angeordnet ist, dass durch die Öffnung (12) von außen in den Behälter (11) einfallendes Licht auf eine Flüssigkeitsoberfläche (22) einer im Behälter (11) aufgenommenen Flüssigkeit (20) gelangen kann,
**dadurch gekennzeichnet, dass** die Vorrichtung (10) wenigstens eine Vibrationseinrichtung (30) umfasst, welche zur Erzeugung von Turbulenz in der im Behälter (11) aufgenommenen Flüssigkeit (20) angeordnet und eingerichtet ist.

2. Vorrichtung (10) zur Produktion phototropher Organismen nach Anspruch 1, **dadurch gekennzeichnet, dass** die Vibrationseinrichtung (30) zur Übertragung von Vibrationen auf die Flüssigkeit (20) eingerichtet ist, insbesondere mittels eines zur Übertragung von Vibrationen auf die Flüssigkeit (20) mechanisch mit der Vibrationseinrichtung (30) gekoppelten Übertragungselements (32) und/oder unter Verwendung eines maschinellen Antriebs (34).

3. Vorrichtung (10) zur Produktion phototropher Organismen nach einem der Ansprüche 1 und 2, **dadurch gekennzeichnet, dass** der Behälter (11) einen im Wesentlichen ebenen Behälterboden (14) mit einer zur Behälterinnenseite weisenden Oberfläche (16) aufweist und die Vorrichtung (10) eine Aufstelleinrichtung zum Aufstellen des Behälters (11) aufweist, die dazu eingerichtet ist, den Behälter (11) derart auf einer Aufstellebene zu positionieren, dass die zur Behälterinnenseite weisende Oberfläche (16) parallel zur Aufstellebene oder in einem Winkel zwischen 0,1° und 5° und insbesondere zwischen 0,5° und 3° in Bezug zur Aufstellebene angeordnet ist.

4. Vorrichtung (10) zur Produktion phototropher Organismen nach wenigstens einem der Ansprüche 1-3, **dadurch gekennzeichnet, dass** der Behälter (11) einen im Wesentlichen ebenen Behälterboden (14) sowie wenigstens ein Wandungselement (18) zwecks Rückhalts der Flüssigkeit (20) im Behälter (11) aufweist, wobei das Wandungselement (18) eine rechtwinklig zum Behälterboden (14) gemessene Höhe H aufweist, die zwischen 0,5 cm und 15 cm und insbesondere zwischen 1 cm und 5 cm beträgt.

5. Vorrichtung (10) zur Produktion phototropher Organismen nach Anspruch 2, **dadurch gekennzeichnet, dass** zumindest ein Teil des Behälters (11) als Übertragungselement (32) ausgestaltet ist, wobei insbesondere der Behälter (11) einen Behälterboden (14) aufweist und der Behälterboden (14) als Übertragungselement (32) ausgestaltet ist.

6. Vorrichtung (10) zur Produktion phototropher Organismen nach wenigstens einem der Ansprüche 1-5, **dadurch gekennzeichnet, dass** diese weiterhin einen Speicherbehälter (40) zum Zwischenspeichern von Flüssigkeit (20) sowie wenigstens eine geeignete Fließverbindung (42) zum Transport von Flüssigkeit (20) vom Behälter (11) in den Speicherbehälter (40) und zurück umfasst.

7. System (50) zur Produktion phototropher Organismen, umfassend eine Vorrichtung (10) zur Produktion phototropher Organismen gemäß wenigstens einem der Ansprüche 1-6, sowie eine darin aufgenommene Flüssigkeit (20), wobei die Flüssigkeit (20) im Wesentlichen Wasser oder im Wesentlichen ein Wasser-Biomasse-Gemisch (52) ist, wobei die Vibrationseinrichtung (30) insbesondere dazu eingerichtet und angeordnet ist, zumindest Teile des Systems (50) zur Schwingung in ihrem Eigenfrequenzbereich ω₀ᵣ oder mit einer Erregerfrequenz fᵣ anzuregen, wobei gilt: fᵣ = ω₀ᵣ * 2ⁿ; wobei n eine ganze Zahl sein soll.

8. Verfahren zur Produktion phototropher Organismen, umfassend die Schritte
∘ Bereitstellung eines Systems (50) zur Produktion phototropher Organismen gemäß Anspruch 7, wobei phototrophe Organismen, insbesondere aquatische Mikroorganismen, sowie insbesondere Nährstoffe in der Flüssigkeit (20) angeordnet sind,
∘ Erzeugen von Turbulenz in der Flüssigkeit (20) mittels der Vibrationseinrichtung (30),
∘ Einbringen von Licht von außen durch die Öffnung (12) des Behälters (11) auf die Flüssigkeitsoberfläche (22).

9. Verfahren zur Produktion phototropher Organismen nach Anspruch 8, **dadurch gekennzeichnet, dass** Kohlendioxid (54) oder ein Kohlendioxid enthaltendes Gasgemisch, insbesondere ein Abgas aus einem Verbrennungsprozess, in die Flüssigkeit (20) eingebracht und/oder auf die Flüssigkeit (20) gegeben wird.

10. Verfahren zur Produktion phototropher Organismen nach einem der Ansprüche 8 und 9, **dadurch gekennzeichnet, dass** wenigstens ein Teil der Flüssigkeit (20) und insbesondere die gesamte Flüssigkeit (20) bewegt wird, so dass sich ein Flüssigkeitsstrom (44) im Behälter (11) einstellt, wobei insbesondere die Geschwindigkeit der Bewegung (46) des Flüssigkeitsstroms (44) maximal 0,5 m/s beträgt.
